(19) Europäisches Patentamt
European Patent Office
Office européen des brevets

(11) Veröffentlichungsnummer: **0 154 269**
**B1**

(12) # EUROPÄISCHE PATENTSCHRIFT

(45) Veröffentlichungstag der Patentschrift: **07.03.90**

(21) Anmeldenummer: **85101988.5**

(22) Anmeldetag: **22.02.85**

(51) Int. Cl.$^5$: **C 12 N 9/86**, C 12 Q 1/34 // (C12N9/86, C12R1:01, 1:06, 1:13, 1:265)

(54) **Nucleosidtriphosphat-abhängige 1-Methylhydantoinase und ihre Verwendung.**

(30) Priorität: **24.02.84 DE 3406770**

(43) Veröffentlichungstag der Anmeldung: **11.09.85 Patentblatt 85/37**

(45) Bekanntmachung des Hinweises auf die Patenterteilung: **07.03.90 Patentblatt 90/10**

(84) Benannte Vertragsstaaten: **AT BE CH DE FR GB IT LI LU NL SE**

(56) Entgegenhaltungen:
**EP-A- 0 112 571**
**US-A- 4 134 793**

(73) Patentinhaber: **BOEHRINGER MANNHEIM GMBH**
**Patentabteilung, Abt. E Sandhofer Strasse 112-132**
**Postfach 31 01 20**
**D-6800 Mannheim 31 Waldhof (DE)**

(72) Erfinder: **Siedel, Joachim, Dr.rer.nat.**
**Bahnhofstrasse 6**
**D-8131 Bernried (DE)**
Erfinder: **Deeg, Rolf, Dr.rer.nat.**
**Hirtenstrasse 7**
**D-8131 Bernried (DE)**
Erfinder: **Röder, Albert, Dr.rer.nat.**
**Tiefentalweg 8**
**D-8124 Seeshaupt (DE)**
Erfinder: **Ziegenhorn, Joachim, Dr.rer.nat.**
**Ina-Seidel-Weg 1**
**D-8130 Starnberg (DE)**
Erfinder: **Möllering, Hans**
**Herrestrasse 10**
**D-8132 Tutzing (DE)**
Erfinder: **Gauhl, Helmgard**
**Mitterfeld 4**
**D-8132 Tutzing (DE)**

(74) Vertreter: **Huber, Bernhard, Dipl.-Chem. et al**
**Patentanwälte H. Weickmann, Dr. K. Fincke F.A. Weickmann, B. Huber Dr. H. Liska, Dr. J. Prechtel**
**Möhlstrasse 22 Postfach 860 820**
**D-8000 München 86 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 154 269 B1

**Beschreibung**

In der analytischen Chemie, insbesondere der klinisch-chemischen Diagnostik besteht ein ständig steigender Bedarf an enzymatischen Verfahren zur Bestimmung von Naturstoffen, biologischen Stoffwechselprodukten und daraus abgeleiteten Verbindungen. Die Gründe sind die außerordentlich hohe Spezifität enzymkatalytischer Substanz-Umsetzungen, ihr rascher, definierter und verlustfreier Ablauf unter milden Reaktionsbedingungen — üblicherweise zwischen 15 und 40 °C im wäßrigen Milieu im neutralen pH-Bereich —, sowie die Möglichkeit, sie auf einfache und empfindliche Weise, insbesondere mit photometrischen Meßmethoden entweder direkt oder mit Hilfe gekoppelter Indikatorreaktionen quantitativ erfassen zu können.

Für 1-Methylhydantoin war bislang kein enzymkatalysiertes Umsetzungsverfahren bekannt, das diese Verbindung einer direkten oder indirekten enzymatischen Analyse zugänglich gemacht hätte. Ein solches Verfahren wäre aber unter anderem besonders wertvoll für die Bestimmung von Creatinin, einem klinisch-diagnostisch bedeutsamen Serum- und Urinbestandteil, der sich in einer seit längerem bekannten Enzymreaktion mittels der Creatinin-Iminohydrolase (E.C. 3.5.4.21) in 1-Methylhydantoin und Ammoniak umwandeln läßt.

Für die enzymatische Bestimmung von Creatinin im Serum oder Urin sind zwar schon mehrere Verfahren bekannt (Wahlefeld, A.W., G. Holz und H.U. Bergmeyer, in H.U. Bergmeyer: Methoden der enzymatischen Analyse, 3. Auflage, Band II, Verlag Chemie, Weinheim 1974, S. 1834-1838 ; Fossati, P., L. Prencipe, and G. Berti, Clinical Chemistry (1983) 29, 1494-1496 ; Tanganelli, E., L. Prenzipe, D. Bassi, S. Cambiaghi, and E. Murador, Clinical Chemistry (1983) 28, 1461-1464) ; sie weisen jedoch allesamt den Nachteil auf, daß sie entweder über Creatin (Wahlefeld et al. ; Fossati et al.) oder Ammoniak (Tanganelli et al.) als Zwischenstufen der Reaktionssequenz verlaufen, Substanzen, die von vorneherein in wechselnden und gegenüber dem Creatinin deutlich ins Gewicht fallenden Konzentrationen in der zu analysierenden Serum- bzw. Urinprobe zugegen sind. Es sind hier also zur Creatinin-Bestimmung Differenzmessungen durch zwei getrennte oder hintereinandergeschaltete Reaktionsansätze erforderlich, einem, in dem zunächst das freie Creatin bzw. Ammoniak bestimmt wird, sowie einem zweiten, bei dem durch Zusatz entweder von Creatinin-Amidohydrolase (E.C. 3.5.2.10) oder Creatinin-Iminohydrolase (= Creatinin-Deiminase) der Anteil des zusätzlich aus Creatinin gebildeten Creatins bzw. Ammoniaks erfaßt wird (« Probe/Probenleerwert-Verfahren » oder « $E_1/E_2$-Verfahren »). Derartige Verfahren sind von der manuellen Durchführung her relativ aufwendig und auch nur sehr beschränkt an automatisierten Analysensystemen einsetzbar, insbesondere wenn für den vollständigen Ablauf der Umsetzungsreaktionen längere Inkubationszeiten notwendig sind. Zwar läßt sich im Prinzip durch geeignete Wahl der Reaktionsbedingungen die Creatininbestimmung mit den bekannten enzymatischen Methoden zur Umgehung der Probenleerwert-Messung im sogenannten kinetischen « fixed-time »-Verfahren durchführen ; dies erfordert allerdings ein sehr genaues Einhalten der Meßzeitpunkte unter definierten Temperaturbedingungen, was mit hinreichender Präzision nur an Analysenautomaten gelingt und umgekehrt die manuelle Handhabung weitgehend ausschließt.

Im Gegensatz zu Creatin oder Ammoniak ist 1-Methylhydantoin (« N-Methylhydantoin », « NMH ») kein natürlicher Bestandteil von Serum oder Urin ; eine über 1-Methylhydantoin als Zwischenprodukt verlaufende Creatinin-Bestimmungsmethode böte somit den erheblichen Vorteil, daß dabei auf eine Probenleerwert-Messung verzichtet werden kann, vorausgesetzt, daß die enzymatische Umsetzung von 1-Methylhydantoin selbst als Indikatorreaktion verwendet werden kann, oder gegebenenfalls nachgeschaltete Indikatorreaktionen ebenfalls nicht über natürlicherweise im Serum oder Urin in signifikanten Konzentrationen vorkommende Substanzen ablaufen.

Es besteht daher der Bedarf an einem Mittel und Verfahren zur enzymatischen Analyse von 1-Methylhydantoin, das dessen quantitative, vorzugsweise photometrische Bestimmung ohne gleichzeitige Miterfassung anderer Serum- oder Urinbestandteile ermöglicht.

Die Aufgabe wird erfindungsgemäß gelöst durch die Auffindung des neuen, bislang nicht bekannten Enzyms 1-Methylhydantoinase, das 1-Methylhydantoin in Gegenwart eines Nukleosidtriphosphats und eines mehrwertigen Metallions hydrolysiert unter Bildung des entsprechenden Nukleosiddiphosphats und eines Hydrolyseproduktes, welches in Gegenwart von N-Carbamoylsarcosinamidohydrolase zu Sarcosin abgebaut wird, erhältlich aus Arthrobacter spec. DSM 2563, DSM 2564, Moraxella spec. DSM 2562, Micrococcus spec. DSM 2565 oder Brevibacterium spec. DSM 2843, die auf N-Methylhydantoin gezüchtet werden. Das Nukleosidtriphosphat ist bevorzugt Adenosin-5'-triphosphat (ATP).

In der Literatur sind zwar schon verschiedentlich enzymatische Hydrolysen von Hydantoinen durch « Hydantoinasen » (Hydropyrimidin-Hydrolase, E.C. 3.5.2.2) aus verschiedenen Quellen beschrieben, jedoch konnte eine Wirksamkeit bisher nur gegenüber unsubstituiertem Hydantoin (Wallach, D.P., and S. Grisolia, J. Biol. Chem. (1957) 226, 227-288) bzw. in 5-Stellung substituierten Hydantoinen (DAS 26 31 048 ; DAS 28 11 303 ; Olivieri, R., E. Fascetti, L. Angelini and L. Degen, Biotechnology and Bioengineering (1981) 23, 2173-2183) gezeigt werden. Darüberhinaus ist für keines der betreffenden Enzyme eine Cofaktor-Abhängigkeit festgestellt worden ; das von Wallach et al. beschriebene Enzym benötigt zur Hydrolyse von Hydantoin z. B. auch keine Zusätze von mehrwertigen Metallionen, in der Arbeit von Olivieri et al. wird sogar auf eine deutliche Hemmung der Hydantoin-Hydrolase in Gegenwart

von Ammoniumchlorid (0,1 mol/l) hingewiesen, während die Aktivität des erfindungsgemäßen Enzyms durch Zusatz von Ammoniumsalzen sogar deutlich gesteigert werden kann.

Vorkommen, Isolierung und Eigenschaften des erfindungsgemäßen Enzyms sowie seine Verwendung zur Bestimmung von 1-Methylhydantoin bzw. Creatinin sind im folgenden näher beschrieben.

Das erfindungsgemäße neue Enzym 1-Methylhydantoinase scheint verbreitet in Mikroorganismen vorzukommen. So konnte es in Mikroorganismen der Gattungen Brevibacterium, Moraxella, Micrococcus und Arthrobacter aufgefunden werden. Beispiele für Stämme dieser Gattungen, in denen ein die Aufarbeitung lohnender Gehalt an dem erfindungsgemäßen Enzym festgestellt wurde, sind Arthrobacter spec. DSM 2563, DSM 2564, Moraxella spec. DSM 2562, Micrococcus spec. DSM 2565 oder Brevibacterium spec. DSM 2843.

Bevorzugt wird daher das erfindungsgemäße Enzym gewonnen, indem man einen der vorstehend aufgeführten Mikroorganismen züchtet und das Enzym aus der Biomasse oder/und dem Kulturmedium isoliert.

Folgende Eigenschaften des neuen Enzyms wurden festgestellt :

1. Molgewicht
a) SDS-Gradientengel-Elektrophorese Mr = 125 000

2. pH-Optimum : pH = 7,8
Aktivität in %, 25 °C

| pH ⟶ | 6,5 | 7,0 | 7,5 | 8,0 | 8,5 | 9,0 |
|---|---|---|---|---|---|---|
| TES-Puffer 150 mmol/l | 5 | 76 | 100 | 100 | 95 | 69 |
| TRIS-Puffer 150 mmol/l | 1 | 26 | 60 | 52 | 38 | 19 |

3. Spezifität :
Relative Aktivität in % (über ADP/Pyruvat-Kinase/Lactat-Dehydrogenase)

a) Substrat (jeweils 0,1 mmol/l Endkonzentration im Reaktionsgemisch)
Zur Bestimmung der Substratspezifität wurde wie folgt verfahren :

1. Grundreagenz

| Komponente | Konzentration |
|---|---|
| Kaliumphosphat (pH 8,0) | 75 mmol/l |
| NADH | 0,25 mmol/l |
| ATP | 1,30 mmol/l |
| Phosphoenolpyruvat | 0,42 mmol/l |
| $MgCl_2$ | 2,00 mmol/l |
| Pyruvat-Kinase | 4 U/ml |
| Lactat-Dehydrogenase | 10 U/ml |

2. 1-Methylhydantoinase (15 U/ml in 50 % Glycerin, 20 mmol/l Tris · HCl-Puffer, pH 8,0

3. Hydantoin-Lösungen
Konzentration der Hydantoine (1-Methylhydantoin, Hydantoin, 5-Methylhydantoin sowie 5,5-Dimethylhydantoin) : jeweils 0,6 mmol/l $H_2O$

4. Durchführung des Tests
Wellenlänge 365 nm ; T = 25 °C, d = 1 cm ; Testvolumen 1,21 ml.
In Küvetten pipettieren

(Siehe Tabelle Seite 4 f.)

| | Probe | Reaktionsgemisch-Leerwert |
|---|---|---|
| Grundreagenz (1)<br>Probe (3)<br>Wasser | 1,00 ml<br>0,20 ml | 1,00 ml<br>-<br>0,20 ml |
| mischen, dann hinzufügen | | |
| 1-Methyl-hydantoinase (2) | 0,01 ml | 0,01 ml |
| $\Delta E$ von Probe ($\Delta E_p$) und Reaktionsgemisch-Leerwert ($\Delta E_{RL}$) innerhalb von 2 Minuten nach Zugabe der 1-Methylhydantoinase messen. $\Delta E = \Delta E_p - \Delta E_{RL}$ | | |

5. Berechnung der relativen Reaktionsgeschwindigkeit aus den mit den verschiedenen Hydantoinen ermittelten ΔE/min über ΔE/min (1-Methylhydantoin) = 100 %.

Nachstehende Ergebnisse wurden erhalten :

1-Methylhydantoin

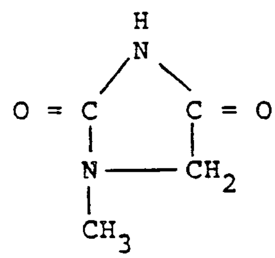

%
100

5-Methylhydantoin

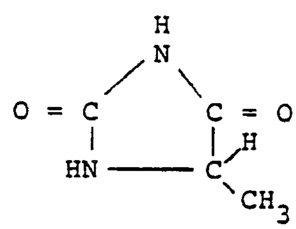

14

Hydantoin

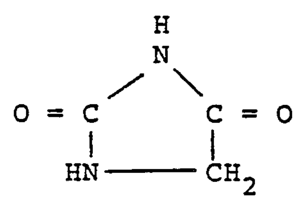

13

5,5-Dimethylhydantoin

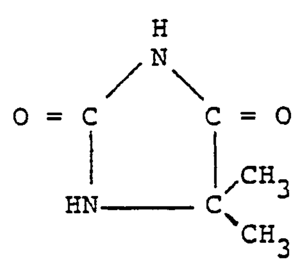

0

b) Nucleotide
Relative Aktivität in % (ATP = 100) bei Bestimmung über Carbamoylsarcosinamidohydrolase + Sarcosinoxidase :

| Nucleotid (4 mmol/l) | % |
|---|---|
| ATP | 100 |
| ADP | 1 |
| AMP | 0 |
| GTP | 7 |
| CTP | 1,5 |
| TTP | 0 |
| UTP | 0 |
| PPi | 0 |
| Carb-Phosphat | 0 |
| Na-Tripolyphosphat | 0 |
| Na-Hexametaphosphat | 0 |

4. Metallionen-Abhängigkeit
Relative Aktivität in % (5 mmol/l Magnesiumchlorid = 100)

| mmol/l | Substanz | % |
|---|---|---|
| 0 | $MgCl_2$ | 17 |
| 1 | " | 83 |
| 5 | " | 100 |
| 10 | " | 98 |
| 5 | $MnCl_2$ | 60 |
| 5 | $ZnCl_2$ | 24 |

5. Aktivatoren
Ausgeprägt $NH_4^+$. Relative Aktivität in % (30 mmol/l AS = 100)

| Aktivator-Substanz | mmol/l | % |
|---|---|---|
| keine | 0 | 44 |
| $(NH_4)_2SO_4$ | 30 | 100 |
| " | 10 | 95 |
| $NH_4Cl$ | 30 | 91 |
| $NH_4$-Acetat | 30 | 98 |
| $Li_2SO_4$ | 30 | 40 |
| $Na_2SO_4$ | 30 | 39 |
| $K_2SO_4$ | 30 | 59 |
| NaCl | 30 | 41 |
| $NaHCO_3$ | 30 | 40 |

6. Inhibitoren
Ausgeprägt EDTA, NaF.
NaF : 1 × $10^{-2}$ mol/l hemmt zu 100 %

| EDTA-Hemmung: * | mmol/l | relative Aktivität % |
|---|---|---|
| | 0 | 100 |
| | 5 | 100 |
| | 10 | 42 |
| | 50 | 2 |

* in Gegenwart von 7,5 mM $Mg^{++}$ im Reaktionsgemisch

7. Michaeliskonstanten
$K_m$ATP (TES 0,15 mol/l, pH 7,8 ; über Sarcosin) : 0,8 mmol/l ; $K_m$ 1-Methylhydantoin (0,15 mol/l TES, pH 7,8 ; über ADP) : 0,02 mmol/l

8. Stabilität
a) Temperaturstabilität : in 50 % Glycerin ; 20 mmol/l Tris, pH 8,0 (2 U/ml)

| 30 min | Restaktivität in % |
|---|---|
| 25°C | 100 |
| 50°C | 94 |
| 60°C | 70 |
| 70°C | 0 |

b) pH-Stabilität
20 % Glycerin, 10 mmol/l Tris, 0,2 U/ml, + 4 °C, 3 Tage dunkel aufbewahrt (Ausgang = 100 %)

| pH | Restaktivität in % |
|---|---|
| 6,3 | 8 |
| 6,5 | 20 |
| 7,0 | 61 |
| 8,0 | 72 |
| 9,0 | 86 |

Das erfindungsgemäße Enzym kann aus Mikroorganismen, die einen die Aufarbeitung lohnenden Gehalt an 1-Methylhydantoinase enthalten, nach an sich bekannten Methoden zur Reinigung von Enzymen erhalten werden. Zweckmäßig wird der Mikroorganismus aufgeschlossen und das Enzym durch Zusatz von Polyethylenimin gefällt. Man erhält so ein Enzympräparat, welches bereits für analytische Zwecke verwendbar ist. Falls eine Weiterreinigung gewünscht wird, wendet man zweckmäßig eine Ammonsulfatfraktionierung, einen Erhitzungsschritt sowie Chromatographie an.

Der Mikroorganismenaufschluß kann nach bekannten chemischen oder physikalischen Methoden erfolgen, beispielsweise chemisch mittels Lysozym oder physikalisch durch Ultraschall, Desintegration in der Hochdrucksuspension und dergleichen. Das Aufschlußverfahren ist nicht kritisch. Besonders geeignet sind jedoch solche Methoden, welche die Zellmembran weitgehend auflösen.

Der Erhitzungsschritt erfolgt zweckmäßig bei 50 °C in einem pH-Bereich, bei dem das Enzym gute Stabilität aufweist.

Für die chromatographische Reinigung haben sich insbesondere Phenylsepharose und DEAE-Gruppen aufweisende schwach basische Anionenaustauscher bewährt. Ein bevorzugtes

Reinigungsverfahren besteht in einem Lysozymaufschluß in 20 %igem Glycerin, Fällung durch Zusatz von löslichem Polyethylenimin, Gewinnung des Niederschlags, Ammonsulfatfraktionierung desselben, Durchführung einer Erhitzungsbehandlung bei 50 °C und pH 8, Chromatographie über Phenylsepharose, gefolgt von Chromatographie über DEAE-Sephacel® und anschließende Molekularsieb-Fraktionierung, zweckmäßig über Sephacryl® S 200. Auf diese Weise erhält man ein reines Enzympräparat mit einer spezifischen Aktivität von 2,1 U/mg Protein, an welchem man die Eigenschaften des Enzyms bestimmen kann.

Die Mikroorganismen für die Gewinnung des Enzyms werden zweckmäßig auf einem Nährmedium kultiviert, welches 1-Methylhydantoin als Kohlenstoff- und Stickstoff-Quelle neben Vitaminen und Spurenmetallen enthält.

Ein weiterer Gegenstand der Erfindung ist die Verwendung der 1-Methylhydantoinase zur Bestimmung von Creatinin in wässriger, gepufferter Lösung durch Überführung des Creatinins mit Creatinin -Deiminase E.C. 3.5.4.21 in 1-Methylhydantoin, Hydrolyse des letzteren mit der 1-Methylhydantoinase in Gegenwart von vorzugsweise im Überschuß vorliegendem Nucleosidtriphosphat und einem mehrwertigen Metallion und Bestimmung

a) des aus 1-Methylhydantoin gebildeten Hydrolyseproduktes mit N-Carbamoylsarcosinamidohydrolase unter Bildung von Sarcosin und Nachweis des Sarcosins mit Sarcosinoxidase oder Sarcosindehydrogenase oder

b) des gleichzeitig gebildeten Nucleosiddiphosphats.

Das entstehende Hydrolyseprodukt verhält sich bezüglich Umsetzungsgeschwindigkeit und Meßsignalhöhe in der nachgeschalteten Indikatorreaktion mit N-Carbamoylsarcosinamidohydrolase praktisch genauso wie dem Reaktionsansatz statt 1-Methylhydantoin in äquimolarer Menge zugesetztes N-Carbamoylsarcosin. (S. auch Fig. 2 und 3).

Der pH-Wert der gepufferten wäßrigen Lösung liegt zweckmäßig zwischen pH 7,0 und 9,0, bevorzugt zwischen pH 7,3 und pH 8,5, insbesondere zwischen pH 7,5 und pH 8,2. Zum Einstellen des pH-Wertes kommen Substanzen in Frage, die im genannten pH-Bereich eine ausreichende Pufferkapazität aufweisen, wie z. B. Phosphat, Tris, Triethanolamin oder TES. Bevorzugt wird insbesondere TES-Puffer. Die Konzentration des Puffers liegt normalerweise zwischen 10 und 500 mmol/l, vorzugsweise zwischen 50 und 200 mmol/l, ganz besonders bevorzugt zwischen 75 und 125 mmol/l.

Als Nucleosidtriphosphat kommen in Frage ATP oder GTP; bevorzugt wird ATP. Der Konzentrationsbereich liegt dabei zweckmäßigerweise zwischen 0,05 bis 50 mmol/l, vorzugsweise zwischen 0,5 bis 20 mmol/l, besonders bevorzugt zwischen 1 bis 10 mmol/l.

Als mehrwertige Metallionen werden bevorzugt zweiwertige Metallionen eingesetzt; besonders bevorzugt werden Mg- und Mn-Ionen, insbesondere Mg-Ionen in Form ihrer wasserlöslichen Salze wie $MgCl_2$ oder $MgSO_4$ oder auch Mg-Aspartat. Die Konzentration an Metallionen liegt dabei zweckmäßig zwischen 0,1 und 50 mmol/l, vorzugsweise zwischen 0,5 und 20 mmol/l, besonders bevorzugt zwischen 1 und 10 mmol/l.

Pro ml Reaktionsgemisch werden normalerweise zwischen 0,01 und 10 U 1-Methylhydantoinase eingesetzt, insbesondere zwischen 0,05 und 2 U, bevorzugt 0,1 bis 1 U.

Zur Aktivitätssteigerung der 1-Methylhydantoinase haben sich Zusätze von Ammoniumsalzen als günstig erwiesen; die Konzentrationen liegen dabei günstigerweise zwischen 0,1 bis 100 mmol/l, bevorzugt zwischen 1 bis 30 mmol/l, insbesondere zwischen 5 und 10 mmol/l.

Die Creatinin-Iminohydrolase wird zweckmäßig mit 0,1 bis 20 U/ml, vorzugsweise 0,05 bis 15 U/ml, insbesondere 1 bis 10 U/ml eingesetzt.

Für die N-Carbamoylsarcosinamidohydrolase sind Aktivitätskonzentrationen von 0,1 bis 20 U/ml günstig, bevorzugt 0,5 bis 10 U/ml, insbesondere 1 bis 5 U/ml. Im übrigen gelten hier die Angaben der DE-A-3248145 analog.

Für die Sarcosin-Oxidase beträgt der günstige Bereich 1 bis 20 U/ml, bevorzugt 2 bis 10 U/ml, besonders bevorzugt 4 bis 8 U/ml. Gleiches gilt für die alternativ einsetzbare Sarcosin-Dehydrogenase.

Der Nachweis des Sarcosins mit Sarcosin-Oxidase oder Sarcosindehydrogenase ist bekannt und die hierfür angegebenen und dem Fachmann bekannten Bedingungen eignen sich auch im Rahmen der Erfindung. Gleiches gilt für den Nachweis des gebildeten Nucleosiddiphosphates, also des ADP oder GDP. Auch hierfür können die bekannten Verfahren bzw. Reagenzien verwendet werden.

Zum Nachweis von gebildetem Sarcosin wird die Verwendung der bekannten Sarcosin-Oxidase-Reaktion besonders bevorzugt. Der Ablauf der Sarcosin-Oxidation kann entweder elektrochemisch über den $O_2$-Verbrauch oder die $H_2O_2$-Bildung im Reaktions-Medium verfolgt werden, oder, bevorzugt, enzymatisch über die Bildung von $H_2O_2$ oder Formaldehyd. Besonders bevorzugt erfolgt die Bestimmung von $H_2O_2$ photometrisch und zwar insbesondere in einer Peroxidase-katalysierten Farbreaktion durch oxidative Kupplung von 2,4,6-Tribrom-3-hydroxybenzoesäure mit 4-Aminoantipyrin. Die Peroxidase-Aktivität beträgt dabei 0,05 bis 20 U/ml, bevorzugt 0,2 bis 10 U/ml, insbesondere 0,5 bis 5 U/ml. Tribromhydroxybenzoesäure wird in Konzentrationen von 1 bis 25 mmol/l, bevorzugt von 2 bis 20 mmol/l, insbesondere von 5 bis 10 mmol/l eingesetzt. Für 4-Aminoantipyrin haben sich Konzentrationen von 0,1 bis 2 mmol/l, bevorzugt von 0,2 bis 1,5 mmol/l, insbesondere von 0,5 bis 1 mmol/l als günstig erwiesen.

Andere bekannte Systeme zum Nachweis von $H_2O_2$ oder, alternativ, von Formaldehyd, können jedoch gleichfalls verwendet werden.

Da die 1-Methylhydantoinase Nucleosidtriphosphat, stöchiometrisch in Nucleosiddiphosphat überführt, kann der Creatininbestimmung anstelle der Bestimmung des aus dem 1-Methylhydantoin gebildeten Reaktionsproduktes auch die Bestimmung des gleichzeitig gebildeten Nucleosiddiphosphates, also insbesondere von ADP, zugrunde gelegt werden. Geeignete Verfahren zur Bestimmung von ADP sind bekannt, beispielsweise aus H.U. Bergmeyer « Methoden der enzymatischen Analyse » 3. Auflage, 1974, Seiten 2128 ff und 2178 ff. Eine detaillierte Beschreibung erübrigt sich daher an dieser Stelle.

Ein weiterer Gegenstand der Erfindung ist ein Reagenz zur Bestimmung von Creatinin, welches gekennzeichnet ist durch einen Gehalt an 1-Methylhydantoinase.

Vorzugsweise enthält dieses Reagenz Creatinin-Deiminase, 1-Methylhydantoinase, ATP oder GTP, zweiwertige Metallionen, N-Carbamoylsarcosinamidohydrolase, Sarcosinoxidase oder Sarcosindehydrogenase und Puffersubstanz pH 7,0 bis 9,0.

Das Reagenz enthält vorzugsweise zusätzlich auch ein System zur Bestimmung von $H_2O_2$ oder von Formaldehyd oder ein farbgebendes Elektronenakzeptorsystem wie z. B. ein Tetrazoliumsalz zur direkten Visualisierung der Sarcosindehydrogenase-Reaktion. Diese Systeme sind bekannt, z. B. aus H.U. Bergmeyer, Methoden der enzymatischen Analyse, Verlag Chemie, Weinheim.

Neben den genannten Bestandteilen kann das Reaktionsgemisch noch Hilfsstoffe wie Konservierungsmittel, z. B. Natriumazid, Detergenzien und Lipasen zur Aufklarung von durch Triglyceride getrübten Proben, sowie Kalium-Ferrocyanid und Ascorbat-Oxidase zur Beseitigung von durch Bilirubin oder Ascorbinsäure in der Probe hervorgerufenen Störungen der $H_2O_2$-Nachweisreaktion enthalten. Zur Verwendung des Reaktionsgemisches zur Bestimmung von 1-Methylhydantoin alleine kann die Creatinin-Iminohydrolase auch weggelassen werden.

Die erfindungsgemäßen Reagenzien enthalten als zweiwertige Metallionen zweckmäßig insbesondere Magnesium- oder Manganionen, wie oben schon näher erläutert.

Die genannten Reaktionskomponenten können auch auf porösen Trägermaterialien imprägniert vorliegen und damit z. B. die quantitative oder qualitative Bestimmung von Creatinin bzw. 1-Methylhydantoin mit sogenannten Streifentests ermöglichen.

In einer anderen bevorzugten Ausführungsform enthält das erfindungsgemäße Reagenz Creatinindeiminase, 1-Methylhydantoinase, ATP oder GTP, zweiwertige Metallionen, insbesondere $Mg^{++}$ oder $Mn^{++}$-Ionen, ein System zur Bestimmung von ADP oder GDP und Puffersubstanz pH 7,0 bis 9,0.

Als Puffersubstanz wird in diesem Fall Kalium-Phosphat-Puffer bevorzugt ; für die Konzentrationen an Puffersubstanz, Creatinindeiminase, 1-Methylhydantoinase, ATP, $Mg^{++}$- oder $Mn^{++}$-Ionen sowie gegebenenfalls Ammoniumsalz gelten im übrigen die oben gemachten Angaben analog.

Der ADP-Nachweis erfolgt vorzugsweise mit Hilfe der gekoppelten Pyruvat-Kinase/Lactat-Dehydrogenase-Reaktion in Gegenwart von Phosphoenolpyruvat und NADH, wobei die gebildete ADP-Menge letztlich über den NADH-Verbrauch in der Lactat-Dehydrogenase-Reaktion photometrisch erfaßt wird. Dieses Verfahren ist dem Fachmann bekannt und bedarf daher keiner näheren Erläuterung an dieser Stelle.

Zur Verwendung des Reaktionsgemisches zur Bestimmung von 1-Methylhydantoin alleine kann die Creatinin-Iminohydrolase auch weggelassen werden.

Die folgenden Beispiele erläutern die Erfindung weiter in Verbindung mit der Zeichung. In dieser stellen dar :

Fig. 1 eine graphische Darstellung der Abhängigkeit der Extinktionswerte, erhalten nach Beispiel 5, von der eingesetzten 1-Methylhydantoin-Menge,

Fig. 2 eine Darstellung gemäß Fig. 1 bei Verwendung von N-Carbamoylsarcosin statt 1-Methylhydantoin in Beispiel 6.

Fig. 3 eine graphische Darstellung, welche die Ergebnisse der Beispiele 5 und 6 gegenüberstellt.

Fig. 4 eine Darstellung gemäß Fig. 1 für Beispiel 7

Fig. 5 eine graphische Darstellung der stöchiometrischen ADP-Bildung

Fig. 6 eine Darstellung gemäß Fig. 1 für Beispiel 8.

Folgende Abkürzungen und Synonyma werden verwendet :

AS : Ammoniumsulfat

Carb-Phosphat : Carbamyl-phosphat

CSHase : N-Carbamoylsarcosin-Amidohydrolase

Creatinindeiminase : Creatinin-Iminohydrolase

1-Methylhydantoinase, NMHase : 1-Methylhydantoin-Hydrolase

N-Methylhydantoin, NMH : 1-Methylhydantoin

OD : Optische Dichte

PABS : Para-aminobenzoesäure

$PP_i$ : Pyrophosphat

TES : 2-{[Tris-(hydroxymethyl)methyl]}amino ethansulfonsäure

TRIS : Tris(hydroxymethyl)aminomethan

Beispiel 1

A) Züchtung des Ausgangsstammes

Arthrobacter species DSM 2563 (isoliert aus Ackererde) wird bei 3-wöchentlicher Überimpfung auf Schrägagar folgender Zusammensetzung gehalten :

pro 1 :

7 g $Na_2HPO_4 \cdot 2\ H_2O$ — 3 g $KH_2PO_4$ 0,5 g NaCl — 0,5 g $MgSO_4 \cdot 7\ H_2O$ — 10 g N-Methylhydantoin (NMH) — 1 ml Spurenlösung 1* — 0,1 ml Spurenlösung 2** — 1 ml Vitaminlösung*** — 20 g Agar
pH 7,5

* Spurenlösung 1
100 mg $MnCl_2 \cdot 4\ H_2O$ — 100 mg $FeCl_3 \cdot 6\ H_2O$ — 100 mg $CaCl_2 \cdot 2\ H_2O$ in 100 ml bidest. Wasser lösen und sterilisieren.
1 ml dieser Lösung auf 1 l Medium.

** Spurenlösung 2
1 mg $CuCl_2 \cdot 2\ H_2O$ — 1 mg $ZnCl_2$ — 1 mg $(NH_4)_2\ MoO_4$ — 1 mg $CoCl_2 \cdot 6\ H_2O$ in 1 000 ml bidest.
Wasser lösen und sterilisieren.
0,1 ml dieser Lösung auf 1 l Medium.

*** Vitaminlösung
0,1 mg Biotin — 0,1 mg Pyridoxol — 0,1 mg Pyridoxamin · HCl — 0,1 mg PABS — 1,0 mg Riboflavin — 1,0 mg Nicotinamid — 1,0 mg Folsäure — 10,0 mg Thiamin · HCl in 100 ml bidest. Wasser lösen und steril filtrieren.
1 ml dieser Lösung auf 1 l Medium.

10 ml des flüssigen, oben beschriebenen Mediums, werden mit 1 Öse voll Material von Schrägagar beimpft und im 100 ml Erlenmeyer 20 bis 24 Stunden bei 28 °C geschüttelt. Anschließend werden diese 10 ml (= 1. Vorkultur) in 1 000 ml des gleichen Mediums überführt und zu je 200 ml/l 1-Erlenmeyer 24 Stunden/28 °C weitergezüchtet (OD 1 : 20 = 0,425). Diese 2. Vorkultur wird anschließend zu 1 % in 100 l des gleichen Mediums in einen Fermenter weitergeimpft und 18 Stunden bei 28 °C fermentiert.

Maximale Enzymsynthese findet am Ende des Wachstums (späte Log-Phase) statt. Aus dem oben angeführten Ansatz werden 1 180 g Feuchtmasse mit 100 U/l NMHase (1-Methylhydantoinase) und 160 U/l CSHase (N-Carbamoylsarcosinamidohydrolase) geerntet.

(Siehe Tabelle Seite 10 f.)

B) Isolierung und Reinigung des Enzyms

Aus 280 g Feuchtmasse (= 20 l Kultur) Arthrobacter wurden 500 U NMHase isoliert:

| Schritt | Vol (ml) | Units | U/mg Prot. | Ausbeute % |
|---|---|---|---|---|
| Lysozym-Aufschluß in 20 % Glycerin | 1400 | 1600 | 0,06 | 100 |
| Polyethylenimin-fällung Niederschlag | 430 | 1586 | - | 99 |
| Ammonsulfat-Fraktionierung = Niederschlag | 120 | 1181 | 0,40 | 73 |
| Erhitzung auf 50°C bei pH 8 Überstand | 90 | 934 | 0,67 | 58 |
| Phenyl-Sepharose-Chromatographie Eluat nach Einengen | 60 | 830 | 1,20 | 52 |
| DEAE-Sephacel-Chromatographie | 8 | 553 | 1,40 | 35 |
| Molekularsieb-Fraktionierung Sephacryl S200 | 10 | 492 | 2,20 | 30,7 |

Endpräparat:
Daten: 22,3 U/ml
10,5 mg/ml
2,1 U/mg Protein
50 % Glycerin
50 mmol/l TRIS,
pH = 8,3.

Die Aktivitätsbestimmung wurde folgendermaßen durchgeführt:

1. Farbreagenz**

| Komponente | Konzentration |
|---|---|
| TES · KOH-Puffer (pH 7,8) | 162 mmol/l |
| 4-Aminoantipyrin | 0,81 mmol/l |
| 2,4,6-Tribrom-3-hydroxybenzoesäure | 8,1 mmol/l |
| $MgCl_2$ | 8,1 mmol/l |
| ATP | 4,3 mmol/l |
| $(NH_4)_2SO_4$ | 32 mmol/l |
| CSHase | 1,1 U/ml |
| Sarcosin-Oxidase | 6,5 U/ml |
| Peroxidase | 2,7 U/ml |

2. 1-Methylhydantoin-Lösung 10 mmol/l

3. Durchführung der Bestimmung
Wellenlänge 546 nm; T = 25 °C; Schichtdicke 1 cm;
Testvolumen 2,03 ml; Extinktionskoeffizient des gebildeten Farbstoffs = 13 $cm^2/\mu mol$.

** Für die Untersuchung nach Punkt 2, Seite 10, 3b, Seite 12 sowie 4, 5 und 6, Seite 13, wurden alternativ die entsprechenden Substanzkonzentrationen oder die Art der zugesetzten Substanzen geändert.

| In Küvette pipettieren | |
|---|---|
| Farbreagenz (1)<br>Probe * | 1,88 ml<br>0,05 ml |
| mischen, abwarten bis gegebenenfalls in der Probe enthaltenes Sarcosin und N-Carbamoylsarcosin abreagiert sind, dann starten mit | |
| 1-Methylhydantoin (2) | 0,10 ml |
| mischen, Extinktionsanstieg verfolgen und ΔE/min aus dem linearen Bereich ermitteln. | |

* (Probenmaterial): Für die Bestimmung von Rohextrakten Überstand des hochtourig zentrifugierten Ultraschall-Aufschlusses der Mikroorganismen in 20 % Glycerin, enthaltend Tris · HCl (pH 8,0), 20 mmol/l sowie 0,1 % Triton X-100, verwenden.

4. Berechnung

$$\text{U/ml Probe*} = \frac{\Delta E/\text{min} \times 2{,}03}{13 \times 0{,}1} \text{ (ggfs. × Probenverdünnung)}$$

Bemerkung: ΔE/min soll nicht größer sein als 0,06; andernfalls Probe verdünnen. Lag-Phase ca. 5 Minuten.

## Beispiel 2

Moraxella spec. DSM 2562 (isoliert aus einer Rinderstallprobe) wird auf Schrägagar, wie unter Beispiel 1 beschrieben, gehalten und im gleichen Medium wie Arthrobacter species gezüchtet.

Vorkultur

20 ml NMH = Medium/100 ml Erlenmeyer werden von Schrägagar beimpft und 21 Stunden bei 28 °C geschüttelt (OD 1 : 20 = 0,430).

Hauptkultur

8 ml dieser Vorkultur werden in 400 ml NMH-Medium/2 l Erlenmeyer weitergeimpft und bis zu 43 Stunden bei 28 °C unter Schütteln kultiviert. In den über Ultraschall aufgeschlossenen Biomassen wurden folgende Aktivitäten gemessen:

| Stunden kultiv. | OD 1:20 | NMHase U/l |
|---|---|---|
| 24 | 0,415 | 41 |
| 28 | 0,495 | 72 |
| 39 | 0,730 | 57 |

## Beispiel 3

Brevibacterium species, DSM 2843 (isoliert aus einer von Beispiel 2 unterschiedenen Stallprobe wird kultiviert wie Moraxella in Beispiel 2.

Vorkultur

21 h/28 °C, OD 1 : 20 = 0,690

Hauptkultur

| Stunden kultiv. | OD 1:20 | NMHase U/l |
|---|---|---|
| 15 | 0,440 | 26 |
| 24 | 0,550 | 80 |
| 39 | 0,670 | 59 |
| 43 | 0,750 | 46 |

Beispiel 4

Micrococcus spec. DSM 2565 (isoliert aus einer Erdprobe) wird kultiviert wie Moraxella in Beispiel 2.
Vorkultur
21 h/28 °C, OD 1 : 20 = 0,620
Hauptkultur

| Stunden kultiv. | OD 1:20 | NMHase U/l |
|---|---|---|
| 24 | 0,690 | 53 |
| 27,5 | 0,800 | 83 |

Beispiel 5

1. Enzymatischer Farbtest zur Bestimmung von 1-Methylhydantoin (1-Methylhydantoinase/N-Carbamoylsarcosinamidohydrolase/Sarcosin-Oxidase-Reaktion mit Peroxidase/4-Aminoantipyrin/2,4,6-Tribrom-5-hydroxy-benzoesäure-Farbindikator-System)

1.1 Reagenz :

| Komponente | Konzentration im Reagenz |
|---|---|
| TES · KOH (pH 7,8) | 100 mmol/l |
| $MgCl_2$ | 5 mmol/l |
| ATP | 5 mmol/l |
| $NH_4Cl$ | 10 mmol/l |
| 4-Aminoantipyrin | 0,5 mmol/l |
| 2,4,6-Tribrom-3-hydroxy-benzoesäure | 5 mmol/l |
| 1-Methylhydantoinase | 0,15 U/ml |
| N-Carbamoylsarcosinamidohydrolase | 2 U/ml |
| Sarcosin-Oxidase | 5 U/ml |
| Peroxidase | 2 U/ml |

1.2 Durchführung der Bestimmung :
Wellenlänge : 546 nm
Schichtdicke : 10 mm Temperatur : 25 °C
Messung gegen Reagenzleerwert.
In Küvette pipettieren :

| | Probewert (P) | Reagenzleerwert (RL) |
|---|---|---|
| Reagenz 1.1 | 2,00 ml | 2,00 ml |
| Probe *) | 0,10 ml | - |
| Wasser | - | 0,10 ml |
| Mischen, 10 Minuten inkubieren und anschließend Extinktion von P gegen RL messen (ΔE). | | |

*) wäßrige Lösungen von 1-Methylhydantoin, 87,7 bis 1 754 µmol/l.

Die Abhängigkeit von ΔE gegen die jeweilige Konzentration von 1-Methylhydantoin in der eingesetzten Probe ist in Fig. 1 wiedergegeben.

## Beispiel 6

Bestimmung von N-Carbamoylsarcosin mit Reagenz 1.1 aus Beispiel 5

Die Durchführung der Bestimmung erfolgt analog Punkt 1.2 aus Beispiel 5, nur daß statt der wäßrigen 1-Methylhydantoin-Lösungen entsprechende Proben mit äquimolaren Konzentrationen (87,7 bis 1 754 μmol/l) an N-Carbamoylsarcosin im Test eingesetzt werden.

Die Abhängigkeit der gemessenen Extinktionswerte gegen die Konzentration von N-Carbamoylsarcosin ist in Fig. 2 wiedergegeben.

Ein Vergleich zwischen den nach Beispiel 5 und Beispiel 6 gemessenen Extinktionswerten zeigt, daß mit 1-Methylhydantoin- und N-Carbamoylsarcosin-Proben jeweils äquimolarer Konzentrationen identische Farbsignalhöhen gemessen werden (Fig. 3).

## Beispiel 7

Enzymatischer UV-Test zur Bestimmung von 1-Methylhydantoin (1-Methylhydantoinase/Pyruvat-Kinase/Lactat-Dehydrogenase-Reaktion)

7.1 Reagenzien :

7.1.1 Reagenz I :

| Komponente | Konzentration im Reagenz |
|---|---|
| Kaliumphosphat (pH 8,0) | 75 mmol/l |
| NADH | 0,25 mmol/l |
| ATP | 1,30 mmol/l |
| Phosphoenolpyruvat | 0,42 mmol/l |
| $MgCl_2$ | 2,00 mmol/l |
| Pyruvat-Kinase | 4 U/ml |
| Lactat-Dehydrogenase | 10 U/ml |

7.1.2 1-Methylhydantoinase (15 U/ml in 50 % Glycerin pH 8,0)

7.2 Durchführung der Bestimmung
Wellenlänge 365 nm
Schichtdicke : 10 mm
Temperatur : 25 °C
Messung gegen Reaktionsgemisch-Leerwert
In Küvetten pipettieren :

| | Probe (P) | Reaktionsgemisch-Leerwert (RL) |
|---|---|---|
| Reagenz I | 1,00 ml | 1,00 ml |
| Probe **) | 0,20 ml | - |
| Wasser | - | 0,20 ml |

**) wäßrige 1-Methylhydantoin-Lösungen, 87,7 bis 877 μmol/l

(Siehe Tabelle Seite 14 f.)

| |
|---|
| 4 Minuten inkubieren, Ausgangsextinktion von Probe ($E_{1,P}$) und von RL ($E_{1,RL}$) messen. Dann hinzumischen: |

| | | |
|---|---|---|
| NMHase | 0,01 ml | 0,01 ml |

| |
|---|
| Nach weiteren 5 bis 10 Minuten Extinktionen von P ($E_{2,P}$) und RL ($E_{2,RL}$)***) messen. $E = (E_{1,P} - E_{2,P}) - (E_{1,RL} - E_{2,RL})$ |

***) Anmerkung: enthält die 1-Methylhydantoinase-Präparation noch signifikante Mengen an ATPase oder NADH-Oxidase, was sich in einer deutlichen NADH-Abnahme im Reaktionsgemisch-Leerwert-Ansatz nach Zugabe der 1-Methylhydantoinase-Lösung zeigt, müssen $E_{2,P}$ und $E_{2,RL}$ jeweils nach genau gleichen Zeitabständen nach Zumischung der NMHase (z. B. nach 6,00 Minuten) abgelesen werden.

Die Abhängigkeit zwischen den gemessenen Extinktionsdifferenzen (ΔE) und den jeweiligen Konzentrationen von 1-Methylhydantoin in der Probe ist in Fig. 4 dargestellt; aus der Berechnung der bei der Hydrolyse von 1-Methylhydantoin entstandenen ADP-Menge über den molaren Extinktionskoeffizienten von NADH bei 365 nm ergibt sich eine Reaktionsstöchiometrie von ATP-Verbrauch bzw. ADP-Bildung zu 1-Methylhydantoin-Hydrolyse von 1 : 1 (Fig. 5).

Beispiel 8

Enzymatischer Farbtest zur Bestimmung von Creatinin

8.1 Reagenz
Entsprechend Reagenz 1.1 aus Beispiel 5 mit Zusatz von Creatinin-Iminohydrolase, 2 U/ml (Endkonzentration).

8.2 Durchführung der Bestimmung:
Entsprechend Punkt 1.2 aus Beispiel 5, jedoch werden als Probe wäßrige Creatinin-Lösungen, 87,7 bis 1 754 µmol/l eingesetzt und die Inkubationsdauer für P und RL wird auf 20 Minuten verlängert.

Die Abhängigkeit von ΔE gegen die Konzentration von Creatinin in der Probe ist in Fig. 6 aufgetragen.

Wird aus Reagenz 8.1 Creatinin-Iminohydrolase wegelassen, so wird keine Farbreaktion beobachtet, desgleichen, wenn im Testansatz 8.2 statt der Creatinin-Lösungen eine Creatinlösung, 884 µmol/l, als Probe eingesetzt wird.

**Patentansprüche** (für die Vertragsstaaten: BE, CH, DE, FR, GB, IT, LI, LU, NL, SE)

1. 1-Methylhydantoinase, welche 1-Methylhydantoin in Gegenwart eines Nukleosidtriphosphats und eines mehrwertigen Metallions hydrolysiert unter Bildung des entsprechenden Nukleosiddiphosphats und eines Hydrolyseproduktes, welches in Gegenwart von N-Carbamoylsarcosinamidohydrolase zu Sarcosin abgebaut wird, erhältlich aus Arthrobacter spec. DSM 2563, DSM 2564, Moraxella spec. DSM 2562, Micrococcus spec. DSM 2565 oder Brevibacterium spec. DSM 2843, die auf N-Methylhydantoin gezüchtet werden.

2. 1-Methylhydantoinase nach Anspruch 1, gekennzeichnet durch ein Molekulargewicht von etwa 125.000 (SDS-Gradientengel-Elektrophorese), ein pH-Optimum bei pH 7,8, eine $K_M$ gegenüber 1-Methylhydantoin von 0,02 mmol/l und eine $K_M$ gegenüber ATP von 0,8 mmol/l.

3. Verfahren zur Gewinnung des Enzyms von Anspruch 1 oder 2, dadurch gekennzeichnet, daß man Arthrobacter spec. DSM 2563, DSM 2564, Moraxella spec. DSM 2562, Micrococcus spec. DSM 2565 oder Brevibacterium spec. DSM 2843 züchtet und das Enzym isoliert.

4. Verfahren nach Anspruch 3, dadurch gekennzeichnet, daß man den Mikroorganismus aufschließt, das Enzym mit Polyethylamin fällt und gegebenenfalls durch Ammonsulfatfraktionierung, Erhitzungsschritt und Chromatographie anreichert.

5. Verwendung des Enzyms von Anspruch 1 zur Bestimmung von Creatinin durch Überführung des Creatinins mit Creatinin-Deiminase E.C. 3.5.4.21 in 1-Methylhydantoin, Hydrolyse des letzteren mit der 1-Methylhydantoinase in Gegenwart von Nucleosidtriphosphat und einem mehrwertigen Metallion und Bestimmung

a) des aus 1-Methylhydantoin gebildeten Hydrolyseproduktes mit N-Carbamoylsarcosinamidohydrolase unter Bildung von Sarcosin und Nachweis des Sarcosins mit Sarcosinoxidase oder Sarcosindehydrogenase oder

b) des gleichzeitig gebildeten Nucleosiddiphosphats.

6. Verfahren nach Anspruch 5, dadurch gekennzeichnet, daß die Bestimmung in einer gepufferten wäßrigen Lösung bei einem pH-Wert zwischen 7,0 und 9,0 durchgeführt wird.

7. Verfahren nach Anspruch 6, dadurch gekennzeichnet, daß man bei einem pH-Wert zwischen 7,3 und 8,5 umsetzt.

8. Verfahren nach einem der Ansprüche 5 bis 7, dadurch gekennzeichnet, daß man die Umsetzung bei einer Pufferkonzentration zwischen 10 und 500 mmol/l durchführt.

9. Verfahren nach Anspruch 8, dadurch gekennzeichnet, daß man eine Pufferkonzentration zwischen 50 und 200 mmol/l einstellt.

10. Verfahren nach einem der Ansprüche 5 bis 9, dadurch gekennzeichnet, daß man als Nucleosidtriphosphat ATP verwendet.

11. Verfahren nach Anspruch 10, dadurch gekennzeichnet, daß man 0,05 bis 50 mmol/l ATP einsetzt.

12. Verfahren nach einem der Ansprüche 5 bis 11, dadurch gekennzeichnet, daß man als mehrwertige Metallionen Magnesium- oder Manganionen zusetzt.

13. Verfahren nach Anspruch 12, dadurch gekennzeichnet, daß man die Magnesiumionen in Form von Magnesiumchlorid, Magnesiumsulfat oder Magnesiumaspartat zusetzt.

14. Verfahren nach Anspruch 12 oder 13, dadurch gekennzeichnet, daß man 0,1 bis 50 mmol/l zweiwertiges Metallion zusetzt.

15. Verfahren nach einem der Ansprüche 5 bis 14, dadurch gekennzeichnet, daß man 0,01 bis 10 U/ml 1-Methylhydantoinase zusetzt.

16. Verfahren nach einem der Ansprüche 5 bis 15, dadurch gekennzeichnet, daß man 0,1 bis 100 mmol/l eines Ammoniumsalzes zusetzt.

17. Verfahren nach einem der Ansprüche 5 bis 16, dadurch gekennzeichnet, daß man 0,1 bis 20 U/ml N-Carbamoylsarcosin-Amidohydrolase zusetzt.

18. Verfahren nach Anspruch 17, dadurch gekennzeichnet, daß man 1 bis 20 U/ml Sarcosinoxidase oder Sarcosindehydrogenase zusetzt.

19. Reagenz zur Bestimmung von Creatinin, gekennzeichnet durch einen Gehalt an 1-Methylhydantoinase.

20. Reagenz nach Anspruch 19, dadurch gekennzeichnet, daß es Creatinindeiminase, 1-Methylhydantoinase, ATP oder GTP, zweiwertige Metallionen, N-Carbamoylsarcosinamidohydrolase, Sarcosinoxidase oder Sarcosindehydrogenase und Puffersubstanz pH 7,0 bis 9,0 enthält.

21. Reagenz nach Anspruch 20, dadurch gekennzeichnet, daß es ein System nur Bestimmung von $H_2O_2$ oder von Formaldehyd oder ein farbgebendes Elektronenakzeptorsystem zur direkten Visualisierung der Sarcosindehydrogenase-Reaktion enthält.

22. Reagenz nach Anspruch 19, dadurch gekennzeichnet, daß es Creatinindeiminase, 1-Methylhydantoinase, ATP oder GTP, zweiwertige Metallionen, ein System zur Bestimmung von ADP oder GDP und Puffersubstanz pH 7,0 bis 9,0 enthält.

23. Reagenz nach Anspruch 20 bis 22, dadurch gekennzeichnet, daß es Mg- oder Mn-Ionen enthält.

24. Reagenz nach Anspruch 22, dadurch gekennzeichnet, daß das System zur Bestimmung von ADP aus Pyruvatkinase, Lactatdehydrogenase, Phosphonoenolpyruvat und NADH besteht.

**Patentansprüche** (für den Vertragsstaat AT)

1. Verfahren zur Gewinnung von 1-Methylhydantoinase, welche 1-Methylhydantoin in Gegenwart eines Nukleosidtriphosphats und eines mehrwertigen Metallions hydrolysiert unter Bildung des entsprechenden Nukleosiddiphosphats und eines Hydrolyseproduktes, welches in Gegenwart von N-Carbamoylsarcosinamidohydrolase zu Sarcosin abgebaut wird, dadurch gekennzeichnet, daß man Arthrobacter spec. DSM 2563, DSM 2564, Moraxella spec. DSM 2562, Micrococcus spec. DSM 2565 oder Brevibacterium spec. DSM 2843 auf N-Methylhydantoin züchtet und das Enzym isoliert.

2. Verfahren nach Anspruch 1, dadurch gekennzeichnet, daß man den Mikroorganismus aufschließt, daß Enzym mit Polyethylenimin fällt und gegebenenfalls durch Ammonsulfatfraktionierung, Erhitzungsschritt und Chromatographie anreichert.

3. Verwendung des Enzyms 1-Methylhydantoinase, welche 1-Methylhydantoin in Gegenwart eines Nukleosidtriphosphats und eines mehrwertigen Metallions hydrolysiert unter Bildung des entsprechenden Nukleosiddiphosphats und eines Hydrolyseproduktes, welches in Gegenwart von N-Carbamoylsarcosinamidohydrolase zu Sarcosin abgebaut wird zur Bestimmung von Creatinin durch Überführung des Creatinins mit Creatinin-Deiminase E.C. 3.5.4.21 in 1-Methylhydantoin, Hydrolyse des letzteren mit der 1-Methylhydantoinase in Gegenwart von Nucleosidtriphosphat und einem mehrwertigen Metallion und Bestimmung

a) des aus 1-Methylhydantoin gebildeten Hydrolyseproduktes mit N-Carbamoylsarcosinamidohydrolase unter Bildung von Sarcosin und Nachweis des Sarcosins mit Sarcosinoxidase oder Sarcosindehydrogenase oder

b) des gleichzeitig gebildeten Nucleosiddiphosphats.

4. Verfahren nach Anspruch 3, dadurch gekennzeichnet, daß die Bestimmung in einer gepufferten wäßrigen Lösung bei einem pH-Wert zwischen 7,0 und 9,0 durchgeführt wird.

5. Verfahren nach Anspruch 4, dadurch gekennzeichnet, daß man bei einem pH-Wert zwischen 7,3 und 8,5 umsetzt.

6. Verfahren nach einem der Ansprüche 3 bis 5, dadurch gekennzeichnet, daß man die Umsetzung bei einer Pufferkonzentration zwischen 10 und 500 mmol/l durchführt.

7. Verfahren nach Anspruch 6, dadurch gekennzeichnet, daß man eine Pufferkonzentration zwischen 50 und 200 mmol/l einstellt.

8. Verfahren nach einem der Ansprüche 3 bis 7, dadurch gekennzeichnet, daß man als Nucleosidtriphosphat ATP verwendet.

9. Verfahren nach Anspruch 8, dadurch gekennzeichnet, daß man 0,05 bis 50 mmol/l ATP einsetzt.

10. Verfahren nach einem der Ansprüche 3 bis 9, dadurch gekennzeichnet, daß man als mehrwertige Metallionen Magnesium- oder Manganionen zusetzt.

11. Verfahren nach Anspruch 10, dadurch gekennzeichnet, daß man die Magnesiumionen in Form von Magnesiumchlorid, Magnesiumsulfat oder Magnesiumaspartat zusetzt.

12. Verfahren nach Anspruch 10 oder 11, dadurch gekennzeichnet, daß man 0,1 bis 50 mmol/l zweiwertiges Metallion zusetzt.

13. Verfahren nach einem der Ansprüche 3 bis 12, dadurch gekennzeichnet, daß man 0,01 bis 10 U/ml 1-Methylhydantoinase zusetzt.

14. Verfahren nach einem der Ansprüche 3 bis 13, dadurch gekennzeichnet, daß man 0,1 bis 100 mmol/l eines Ammoniumsalzes zusetzt.

15. Verfahren nach einem der Ansprüche 3 bis 14, dadurch gekennzeichnet, daß man 0,1 bis 20 U/ml N-Carbamoylsarcosin-Amidohydrolase zusetzt.

16. Verfahren nach Anspruch 15, dadurch gekennzeichnet, daß man 1 bis 20 U/ml Sarcosinoxidase oder Sarcosindehydrogenase zusetzt.

17. Reagenz zur Bestimmung von Creatinin, gekennzeichnet, durch einen Gehalt an 1-Methylhydantoinase und Creatinindeiminase.

18. Reagenz nach Anspruch 17, dadurch gekennzeichnet, daß es Creatinindeiminase, 1-Methylhydantoinase, ATP oder GTP, zweiwertige Metallionen, N-Carbamoylsarcosinamidohydrolase, Sarcosinoxidase oder Sarcosindehydrogenase und Puffersubstanz pH 7,0 bis 9,0 enthält.

19. Reagenz nach Anspruch 18, dadurch gekennzeichnet, daß es ein System zur Bestimmung von $H_2O_2$ oder von Formaldehyd oder ein farbgebendes Elektronenakzeptorsystem zur direkten Visualisierung der Sarcosindehydrogenase-Reaktion enthält.

20. Reagenz nach Anspruch 17, dadurch gekennzeichnet, daß es Creatinindeiminase, 1-Methylhydantoinase, ATP oder GTP, zweiwertige Metallionen, ein system zur Bestimmung von ADP oder GDP und Puffersubstanz pH 7,0 bis 9,0 enthält.

21. Reagenz nach Anspruch 18 bis 20, dadurch gekennzeichnet, daß es Mg- oder Mn-Ionen enthält.

22. Reagenz nach Anspruch 20, dadurch gekennzeichnet, daß das System zur Bestimmung von ADP aus Pyruvatkinase, Lactadehydrogenase, Phosphonoenolpyruvat und NADH besteht.

**Claims** (for the Contracting States : BE, CH, DE, FR, GB, IT, LI, LU, NL, SE)

1. 1-Methylhydantoinase, which hydrolyses 1-methylhydantoin in the presence of a nucleoside triphosphate and of a polyvalent metal ion with formation of the corresponding nucleoside diphosphate and of a hydrolysis product which is broken down to sarcosine in the presence of N-carbamoylsarcosinamidohydrolase, obtainable from Arthrobacter spec., DSM 2563, DSM 2564, Moraxella spec., DSM 2562, Micrococcus spec., DSM 2565 or Brevibacterium spec., DSM 2843, which are cultured on N-methylhydantoin.

2. 1-Methylhydantoinase according to claim 1, characterised by a molecular weight of about 125,000 (SDS gradient gel electrophoresis), a pH optimum at pH 7.8, a $K_M$ towards 1-methylhydantoin of 0.02 mmol/l. and a $K_M$ towards ATP of 0.8 mmol/l.

3. Process for the obtaining of the enzyme of claim 1 or 2, characterised in that one cultures Arthrobacter spec. DSM 2563, DSM 2564, Moraxella spec. DSM 2562, Micrococcus spec. DSM 2565 or Brevibacterium spec. DSM 2843 and isolates the enzyme.

4. Process according to claim 3, characterised in that one digests the micro-organism, precipitates the enzyme with polyethyleneimine and optionally enriches by ammonium sulphate fractionation, heating step and chromatography.

5. Use of the enzyme of claim 1 for the determination of creatinine by conversion of the creatinine with creatinine deiminase (E.C. 3.5.4.21) into 1-methylhydantoin, hydrolysis of the latter with the 1-methylhydantoinase in the presence of nucleoside triphosphate and of a polyvalent metal ion and determination

a) of the hydrolysis product formed from 1-methylhydantoin with N-carbamoylsarcosineamidohydrolase with formation of sarcosine and detection of the sarcosine with sarcosine oxidase or sarcosine dehydrogenase or

b) of the simultaneously formed nucleoside diphosphate.

6. Process according to claim 5, characterised in that the determination is carried out in a buffered aqueous solution at a pH value between 7.0 and 9.0.

7. Process according to claim 6, characterised in that one reacts at a pH value between 7.3 and 8.5.

8. Process according to one of caims 5 to 7, characterised in that one carries out the reaction at a buffer concentration between 10 and 500 mmol/l.

9. Process according to claim 8, characterised in that one adjusts a buffer concentration between 50 and 200 mmol/l.

10. Process according to one of claims 5 to 9, characterised in that one uses ATP as nucleoside triphosphate.

11. Process according to claim 10, characterised in that one uses 0.05 to 50 mmol/l. of ATP.

12. Process according to one of claims 5 to 11, characterised in that one adds magnesium or manganese ions as polyvalent metal ions.

13. Process according to claim 12, characterised in that one adds the magnesium ions in the form of magnesium chloride, magnesium sulphate or magnesium aspartate.

14. Process according to claim 12 or 13, characterised in that one adds 0.1 to 50 mmol/l. of divalent metal ion.

15. Process according to one of claims 5 to 14, characterised in that one adds 0.01 to 10 U/ml. of 1-methylhydantoinase.

16. Process according to one of claims 5 to 15, characterised in that one adds 0.1 to 100 mmol/l. of an ammonium salt.

17. Process according to one of claims 5 to 16, characterised in that one adds 0.1 to 20 U/ml. N-carbamoylsarcosineamidohydrolase.

18. Process according to claim 17, characterised in that one adds 1 to 20 U/ml. sarcosine oxidase or sarcosine dehydrogenase.

19. Reagent for the determination of creatinine, characterised by a content of 1-methylhydantoinase.

20. Reagent according to claim 19, characterised in that it contains creatinine deiminase, 1-methylhydantoinase, ATP or GTP, divalent metal ions, N-carbamoylsarcosinamidohydrolase, sarcosine oxidase or sarcosine dehydrogenase and buffer substance pH 7.0 to 9.0.

21. Reagent according to claim 20, characterised in that it contains a system for the determination of $H_2O_2$ or of formaldehyde or a colour-giving electron acceptor system for the direct visualisation of the sarcosine dehydrogenase reaction.

22. Reagent according to claim 19, characterised in that it contains creatinine deiminase, 1-methylhydantoinase, ATP or GTP, divalent metal ions, a system for the determination of ADP or GDP and buffer substance pH 7.0 to 9.0.

23. Reagent according to claim 20 to 22, characterised in that it contains Mg or Mn ions.

24. Reagent according to claim 22, characterised in that the system for the determination of ATP consists of pyruvate kinase, lactate dehydrogenase, phosphoenol pyruvate and NADH.

**Claims** (for the Contracting State AT)

1. Process for the obtaining of 1-methylhydantoinase, which hydrolyses 1-methylhydantoin in the presence of a nucleoside triphosphate and of a polyvalent metal ion with formation of a corresponding nucleoside diphosphate and of a hydrolysis product which is broken down in the presence of N-carbamoylsarcosineamidohydrolase, characterised in that one cultures Arthrobacter spec. DSM 2563, DSM 2564, Moraxella spec. DSM 2562, Micrococcus spec. DSM 2565 or Brevibacterium spec. DSM 2843 on N-methylhydantoin and isolates the enzyme.

2. Process according to claim 1, characterised in that one digests the micro-organism, precipitates the enzyme with polyethyleneimine and possibly enriches by ammonium sulphate fractionation, heating step and chromatography.

3. Use of the enzyme 1-methylhydantoinase, which hydrolyses 1-methylhydantoin in the presence of a nucleoside triphosphate and of a polyvalent metal ion with formation of the corresponding nucleoside diphosphate and of a hydrolysis product which is broken down in the presence of N-carbamoylsarcosineamidohydrolase to sarcosine for the determination of creatinine by conversion of the creatinine with creatinine deiminase E.C. 3.5.4.21 into 1-methylhydantoin, hydrolysis of the latter with the 1-methylhydantoinase in the presence of nucleoside triphosphate and a polyvalent metal ion and determination

a) of the hydrolysis product formed from 1-methylhydantoin with N-carbamoylsarcosinamidohydrolase with formation of sarcosine and detection of the sarcosine with sarcosine oxidase or sarcosine dehydrogenase or

b) of the simultaneously formed nucleoside diphosphate.

4. Process according to claim 3, characterised in that the determination is carried out in a buffered aqueous solution at a pH value between 7.0 and 9.0.

5. Process according to claim 4, characterised in that one reacts at a pH value between 7.3 and 8.5.

6. Process according to one of claims 3 to 5, characterised in that one carries out the reaction at a buffer concentration between 10 and 500 mmol/l.

7. Process according to claim 6, characterised in that one adjusts a buffer concentration between 50 and 200 mmol/l.

8. Process according to one of claims 3 to 7, characterised in that one uses ATP as nucleoside triphosphate.

9. Process according to claim 8, characterised in that one uses 0.05 to 50 mmol/l. of ATP.

10. Process according to one of claims 3 to 9, characterised in that one uses magnesium or manganese ions as polyvalent metal ions.

11. Process according to claim 12, characterised in that one adds the magnesium ions in the form of magnesium chloride, magnesium sulphate or magnesium aspartate.

12. Process according to claim 10 or 11, characterised in that one adds 0.1 to 50 mmol/l. of divalent metal ion.

13. Process according to one of claims 3 to 12, characterised in that one adds 0.01 to 10 U/ml. of 1-methylhydantoinase.

14. Process according to one of claims 3 to 13, characterised in that one adds 0.1 to 100 mmol/l. of an ammonium salt.

15. Process according to one of claims 3 to 14, characterised in that one adds 0.1 to 20 U/ml. N-carbamoylsarcosine amidohydrolase.

16. Process according to claim 15, characterised in that one adds 1 to 20 U/ml. sarcosine oxidase or sarcosine dehydrogenase.

17. Reagent for the determination of creatinine, characterised by a content of 1-methylhydantoinase and creatinine deiminase.

18. Reagent according to claim 17, characterised in that it contains creatinine deiminase, 1-methylhydantoinase, ATP or GTP, divalent metal ions, N-carbamoylsarcosinamidohydrolase, sarcosine oxidase or sarcosine dehydrogenase and buffer substance pH 7.0 to 9.0.

19. Reagent according to claim 18, characterised in that it contains a system for the determination of $H_2O_2$ or of formaldehyde or a colour-giving electron acceptor system for the direct visualisation of the sarcosine dehydrogenase reaction.

20. Reagent according to claim 17, characterised in that it contains creatinine deiminase, 1-methylhydantoinase, ATP or GTP, divalent metal ions, a system for the determination of ADP or GDP and buffer substance pH 7.0 to 9.0.

21. Reagent according to claim 18 to 20, characterised in that it contains Mg or Mn ions.

22. Reagent according to claim 20, characterised in that the system for the determination of ADP consists of pyruvate kinase, lactate dehydrogenase, phosphoenol pyruvate and NADH.

**Revendications** (pour les Etats contractants : BE, CH, DE, FR, GB, IT, LI, LU, NL, SE)

1. 1-méthylhydantoı nase, qui hydrolyse la 1-méthylhydantoïne en présence d'un triphosphate de nucléoside et d'un ion métallique polyvalent, avec formation du diphosphate de nucléoside correspondant et d'un produit d'hydrolyse, lequel, en présence de N-carbamoylsarcosine amidohydrolase est décomposé en sarcosine, ladite 1-méthylhydantoinase pouvant être obtenue à partir de Arthrobacter spec. DSM 2563, DSM 2564, Moraxella spec. DSM 2562, Micrococcus spec. DSM 2565 ou Brevibacterium spec. DSM 2843, qui sont cultivés sur de la N-méthylhydantoïne.

2. 1-méthylhydantoïnase selon la revendication 1, caractérisé par un poids moléculaire d'environ 125 000 (électrophorèse sur gel à gradient de SDS), un pH optimum à pH 7,8, un $K_M$ vis-à-vis de la 1-méthylhydantoïne de 0,02 mmol/l et un $K_M$ vis-à-vis de l'ATP de 0,8 mmol/l.

3. Procédé pour l'obtention de l'enzyme de la revendication 1 ou 2, caractérisé en ce que l'on cultive Arthrobacter spec. DSM 2563, DSM 2564, Moraxella spec. DSM 2562, Micrococcus spec. DSM 2565 ou Brevibacterium spec. DSM 2843 et isole l'enzyme.

4. Procédé selon la revendication 3, caractérisé en ce qu'on désagrège le microorganisme, précipite l'enzyme au moyen de polyéthylénimine et enrichit éventuellement par fractionnement au sulfate d'ammonium, étape de chauffage et chromatographie.

5. Utilisation de l'enzyme de la revendication 1 pour la détermination de la créatinine par transformation de la créatinine au moyen de créatinine-déiminase E.C. 3.5.4.21 en 1-méthylhydantoı ne, hydrolyse de cette dernière par la 1-méthylhydantoïnase en présence de triphosphate de nucléoside et d'un ion métallique polyvalent et détermination

a) du produit d'hydrolyse formé à partir de la 1-méthylhydantoïne au moyen de la N-carbamoylsarcosine amidohydrolase avec formation de sarcosine et mise en évidence de la sarcosine au moyen de sarcosine oxydase ou de sarcosine déshydrogénase ou

b) du diphosphate de nucléoside formé en même temps.

6. Procédé selon la revendication 5, caractérisé en ce que la détermination est effectuée dans une solution aqueuse tamponnée à une valeur de pH entre 7,0 et 9,0.

7. Procédé selon la revendication 6, caractérisé en ce qu'on fait réagir à une valeur de pH entre 7,3 et 8,5.

8. Procédé selon l'une des revendications 5 à 7, caractérisé en ce qu'on effectue la réaction à une concentration de tampon entre 10 et 500 mmol/l.

9. Procédé selon la revendication 8, caractérisé en ce qu'on règle à une concentration du tampon entre 50 et 200 mmol/l.

10. Procédé selon l'une des revendications 5 à 9, caractérisé en ce que l'on utilise, en tant que triphosphate de nucléoside, de l'ATP.

11. Procédé selon la revendication 10, caractérisé en ce que l'on met en œuvre 0,05 à 50 mmol/l d'ATP.

12. Procédé selon l'une des revendications 5 à 11, caractérisé en ce qu'on ajoute, en tant qu'ions métalliques polyvalents, des ions magnésium ou manganèse.

13. Procédé selon la revendication 12, caractérisé en ce qu'on ajoute les ions magnésium sous forme de chlorure de magnésium, de sulfate de magnésium ou d'aspartate de magnésium.

14. Procédé selon la revendication 12 ou 13, caractérisé en ce qu'on ajoute 0,1 à 50 mmol/l d'ion métallique bivalent.

15. Procédé selon l'une des revendications 5 à 14, caractérisé en ce qu'on ajoute 0,01 à 10 U/ml de 1-méthylhydantoïnase.

16. Procédé selon l'une des revendications 5 à 15, caractérisé en ce qu'on ajoute 0,1 à 100 mmol/l d'un sel d'ammonium.

17. Procédé selon l'une des revendications 5 à 16, caractérisé en ce qu'on ajoute 0,1 à 20 U/ml de N-carbamoylsarcosine amidohydrolase.

18. Procédé selon la revendication 17, caractérisé en ce qu'on ajoute 1 à 20 U/ml de sarcosine oxydase ou de sarcosine déshydrogénase.

19. Réactif pour la détermination de la créatinine, caractérisé par une teneur en 1-méthylhydantoïnase.

20. Réactif selon la revendication 19, caractérisé en ce qu'il contient de la créatinine déiminase, de la 1-méthylhydantoïnase, de l'ATP ou du GTP, des ions métalliques bivalents, de la N-carbamoylsarcosine amidohydrolase, de la sarcosine oxydase ou de la sarcosine déshydrogénase et de la substance tampon pH 7,0 à 9,0.

21. Réactif selon la revendication 20, caractérisé en ce qu'il contient un système pour la détermination de $H_2O_2$ ou de l'aldéhyde formique ou un système accepteur d'électrons donnant une coloration pour la visualisation directe de la réaction de la sarcosine déshydrogénase.

22. Réactif selon la revendication 19, caractérisé en ce qu'il contient de la créatinine déiminase, de la 1-méthylhydantoïnase, de l'ATP ou du GTP, des ions métalliques bivalents, un système pour la détermination de l'ADP ou du GDP et de la substance tampon pH 7,0 à 9,0.

23. Réactif selon la revendication 20 à 22, caractérisé en ce qu'il contient des ions Mg ou Mn.

24. Réactif selon la revendication 22, caractérisé en ce que le système pour la détermination de l'ADP est constitué de pyruvate kinase, de lactate déshydrogénase, de phosphonoénol pyruvate et de NADH.

**Revendications** (pour l'Etat contractant AT)

1. Procédé pour l'obtention de la 1-méthylhydantoïnase, qui hydrolyse la 1-méthylhydantoïne en présence d'un triphosphate de nucléoside et d'un ion métallique polyvalent, avec formation du diphosphate de nucléoside correspondant et d'un produit d'hydrolyse, lequel, en présence de N-carbamoylsarcosine amidohydrolase est décomposé en sarcosine, caractérisé en ce que l'on cultive Arthrobacter spec. DSM 2563, DSM 2564, Moraxella spec. DSM 2562, Micrococcus spec. DSM 2565 ou Brevibacterium spec. DSM 2843 sur de la N-méthylhydantoïne et isole l'enzyme.

2. Procédé selon la revendication 1, caractérisé en ce qu'on désagrège le microorganisme, précipite l'enzyme au moyen de polyéthylénimine et enrichit éventuellement par fractionnement au sulfate d'ammonium, étape de chauffage et chromatographie.

3. Utilisation de l'enzyme 1-méthylhydantoïnase, qui hydrolyse la 1-méthylhydantoïne en présence d'un triphosphate de nucléoside et d'un ion métallique polyvalent, avec formation du diphosphate de nucléoside correspondant et d'un produit d'hydrolyse, lequel, en présence de N-carbamoylsarcosine amidohydrolase est décomposé en sarcosine pour la détermination de la créatinine par transformation de la créatinine au moyen de créatinine-déiminase E.C. 3.5.4.21 en 1-méthylhydantoïne, hydrolyse de cette dernière par la 1-méthylhydantoïnase en présence de triphosphate de nucléoside et d'un ion métallique polyvalent et détermination.

a) du produit d'hydrolyse formé à partir de la 1-méthylhydantoïne au moyen de la N-carbamoylsarcosine amidohydrolase avec formation de sarcosine et mise en évidence de la sarcosine au moyen de sarcosine oxydase ou de sarcosine déshydrogénase ou

b) du diphosphate de nucléoside formé en même temps.

4. Procédé selon la revendication 3, caractérisé en ce que la détermination est effectuée dans une solution aqueuse tamponnée à une valeur de pH entre 7,0 et 9,0.

5. Procédé selon la revendication 4, caractérisé en ce qu'on fait réagir à une valeur de pH entre 7,3 et 8,5.

6. Procédé selon l'une des revendications 3 à 5, caractérisé en ce qu'on effectue la réaction à une concentration de tampon entre 10 et 500 mmol/l.

7. Procédé selon la revendication 6, caractérisé en ce qu'on règle à une concentration du tampon entre 50 et 200 mmol/l.

8. Procédé selon l'une des revendications 3 à 7, caractérisé en ce que l'on utilise, en tant que triphosphate de nucléoside, de l'ATP.

9. Procédé selon la revendication 8, caractérisé en ce que l'on met en œuvre 0,05 à 50 mmol/l d'ATP.

10. Procédé selon l'une des revendications 3 à 9, caractérisé en ce qu'on ajoute, en tant qu'ions métalliques polyvalents, des ions magnésium ou manganèse.

11. Procédé selon la revendication 10, caractérisé en ce qu'on ajoute les ions magnésium sous forme de chlorure de magnésium, de sulfate de magnésium ou d'aspartate de magnésium.

12. Procédé selon la revendication 10 ou 11, caractérisé en ce qu'on ajoute 0,1 à 50 mmol/l d'ion métallique bivalent.

13. Procédé selon l'une des revendications 3 à 12, caractérisé en ce qu'on ajoute 0,01 à 10 U/ml de 1-méthylhydantoïnase.

14. Procédé selon l'une des revendications 3 à 13, caractérisé en ce qu'on ajoute 0,1 à 100 mmol/l d'un sel d'ammonium.

15. Procédé selon l'une des revendications 3 à 14, caractérisé en ce qu'on ajoute 0,1 à 20 U/ml de N-carbamoylsarcosine amidohydrolase.

16. Procédé selon la revendication 15, caractérisé en ce qu'on ajoute 1 à 20 U/ml de sarcosine oxydase ou de sarcosine déshydrogénase.

17. Réactif pour la détermination de la créatinine, caractérisé par une teneur de 1-méthylhydantoïnase et créatinine déiminase.

18. Réactif selon la revendication 17, caractérisé en ce qu'il contient de la créatinine déiminase, de la 1-méthylhydantoïnase, de l'ATP ou du GTP, des ions métalliques bivalents, de la N-carbamoylsarcosine amidohydrolase, de la sarcosine oxydase ou de la sarcosine déshydrogénase et de la substance tampon pH 7,0 à 9,0.

19. Réactif selon la revendication 18, caractérisé en ce qu'il contient un système pour la détermination de $H_2O_2$ ou de l'aldéhyde formique ou un système accepteur d'électrons donnant une coloration pour la visualisation directe de la réaction de la sarcosine déshydrogénase.

20. Réactif selon la revendication 17, caractérisé en ce qu'il contient de la créatinine déiminase, de la 1-méthylhydantoïnase, de l'ATP ou du GTP, des ions métalliques bivalents, un système pour la détermination de l'ADP ou du GDP et de la substance tampon pH 7,0 à 9,0.

21. Réactif selon la revendication 18 à 20, caractérisé en ce qu'il contient des ions Mg ou Mn.

22. Réactif selon la revendication 20, caractérisé en ce que le système pour la détermination de l'ADP est constitué de pyruvate kinase, de lactate déshydrogénase, de phosphonoénol pyruvate et de NADH.

FIG.1

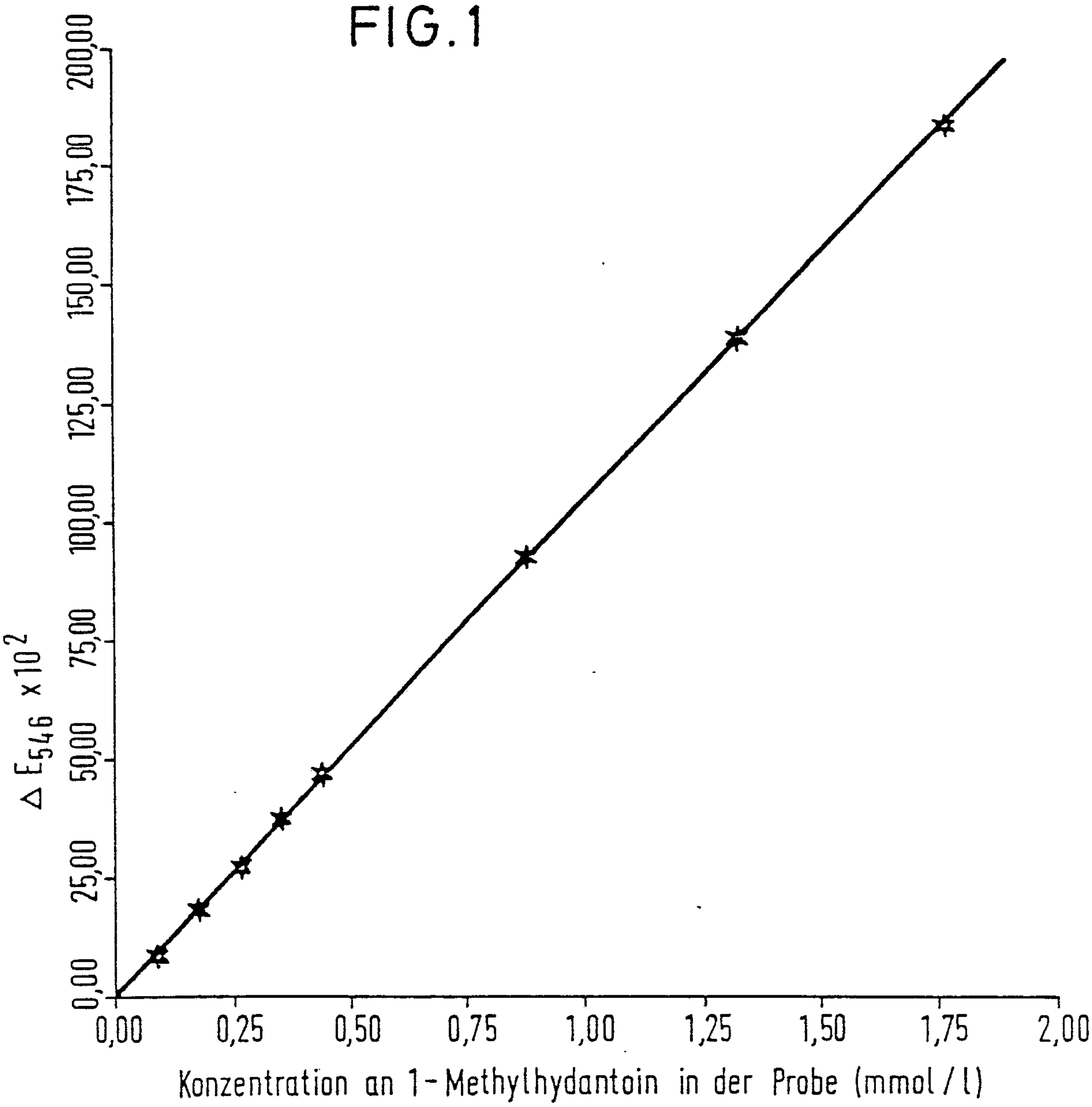
200,00
175,00
150,00
125,00
100,00
75,00
50,00
25,00
0,00
ΔE546 x10²
0,00
0,25
0,50
0,75
1,00
1,25
1,50
1,75
2,00
Konzentration an 1-Methylhydantoin in der Probe (mmol / l)

FIG. 2

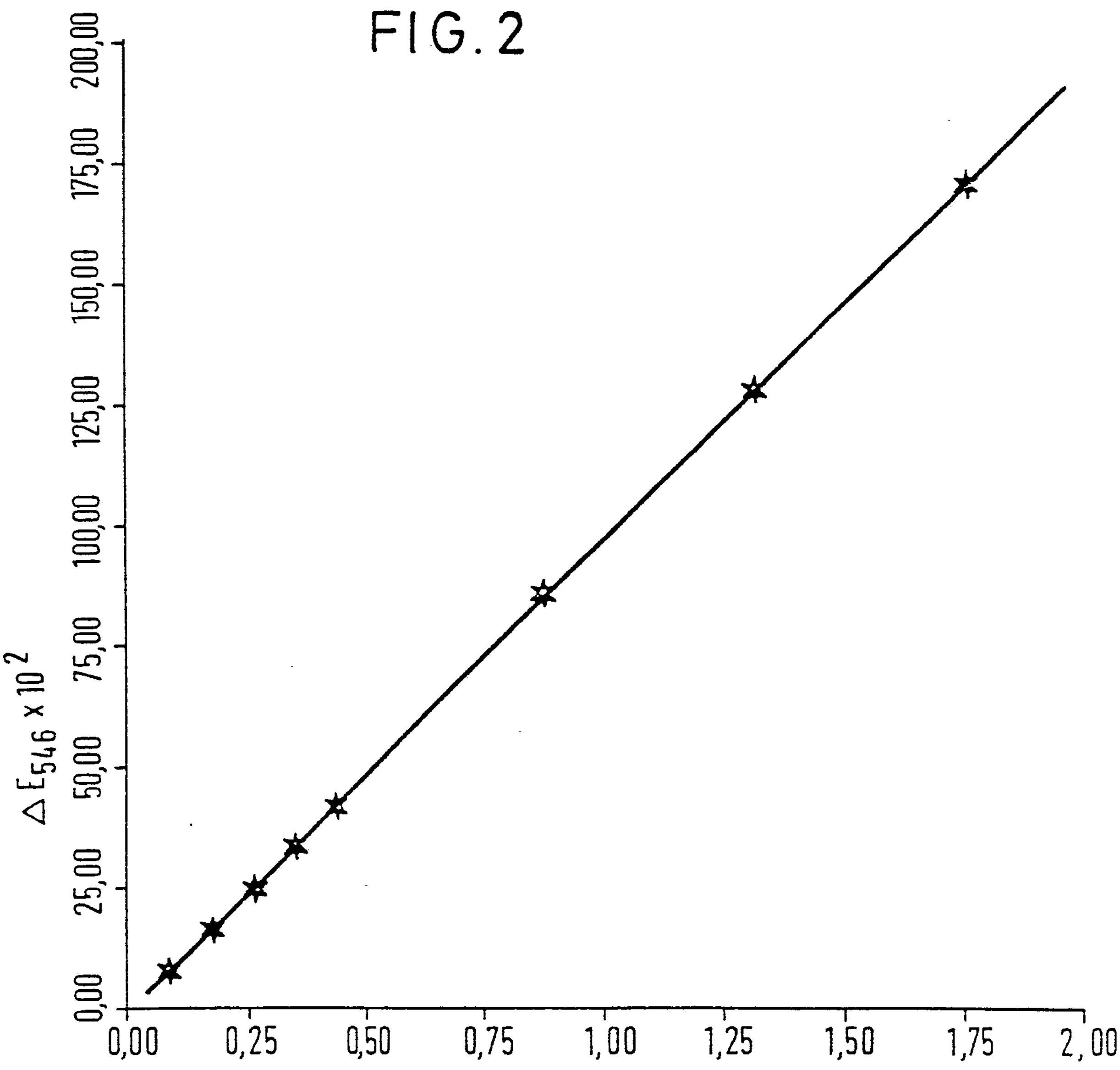
200,00
175,00
150,00
125,00
100,00
75,00
50,00
25,00
0,00
ΔE546 x 10²
0,00
0,25
0,50
0,75
1,00
1,25
1,50
1,75
2,00
Konzentration an N-Carbamoylsarcosin in der Probe (mmol / l)

FIG. 3

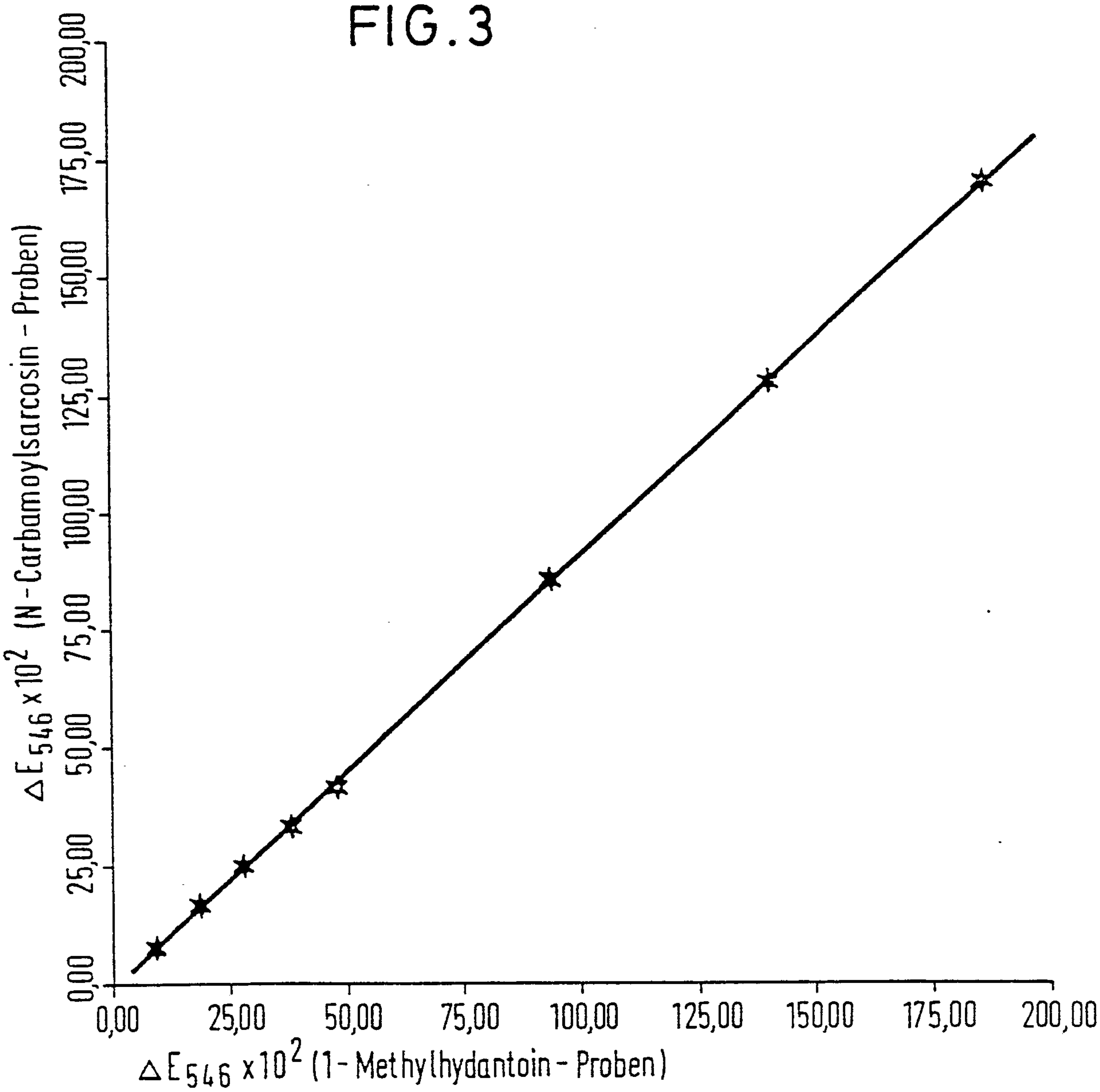
200,00
175,00
150,00
125,00
100,00
75,00
50,00
25,00
0,00
ΔE 546 x10 2 (N-Carbamoylsarcosin - Proben)
0,00 25,00 50,00 75,00 100,00 125,00 150,00 175,00 200,00
ΔE 546 x10 2 (1-Methylhydantoin - Proben)

FIG. 4

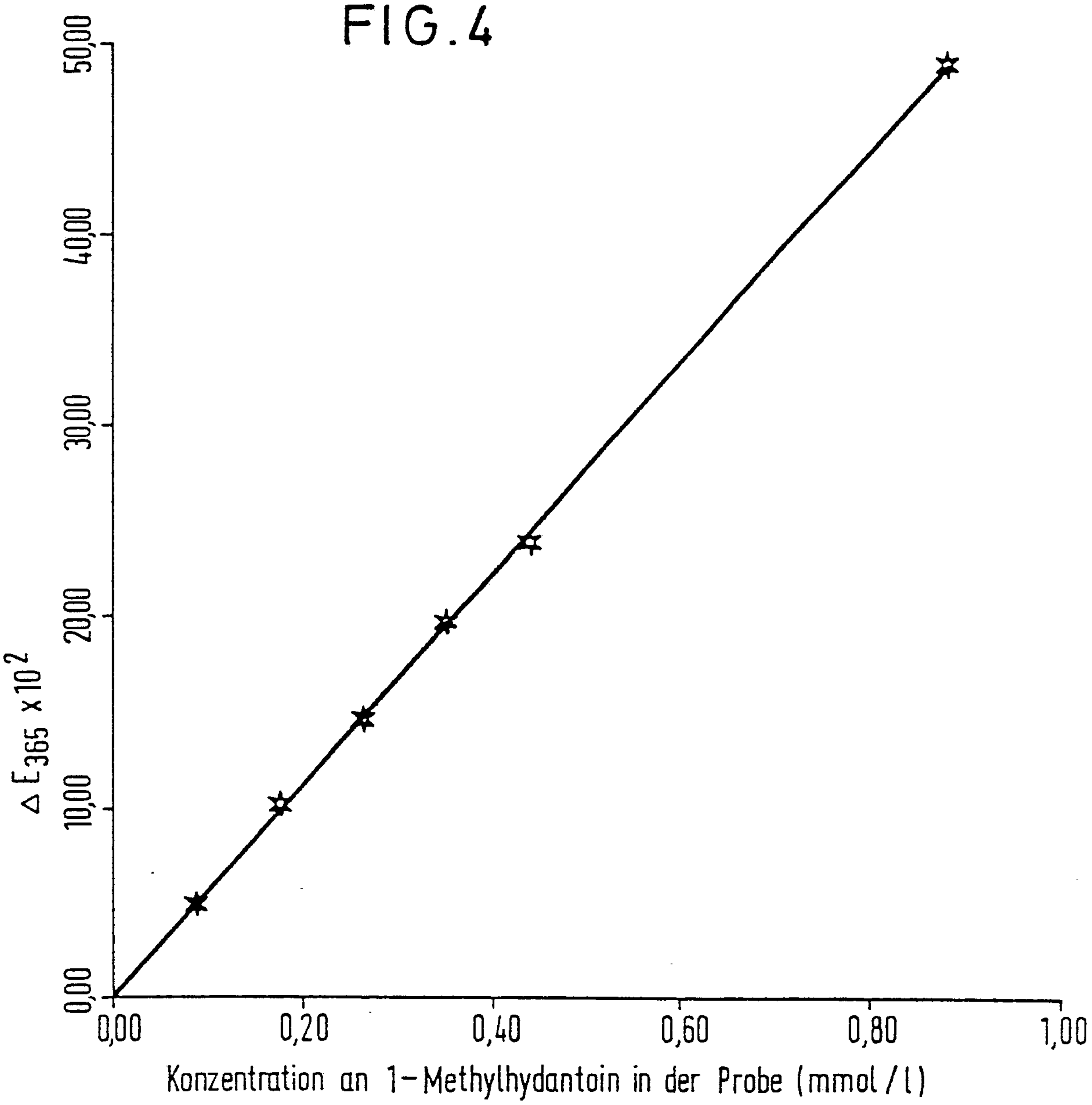
50,00
40,00
30,00
20,00
10,00
0,00
$\Delta E_{365} \times 10^2$
0,00
0,20
0,40
0,60
0,80
1,00
Konzentration an 1-Methylhydantoin in der Probe (mmol / l)

FIG.5

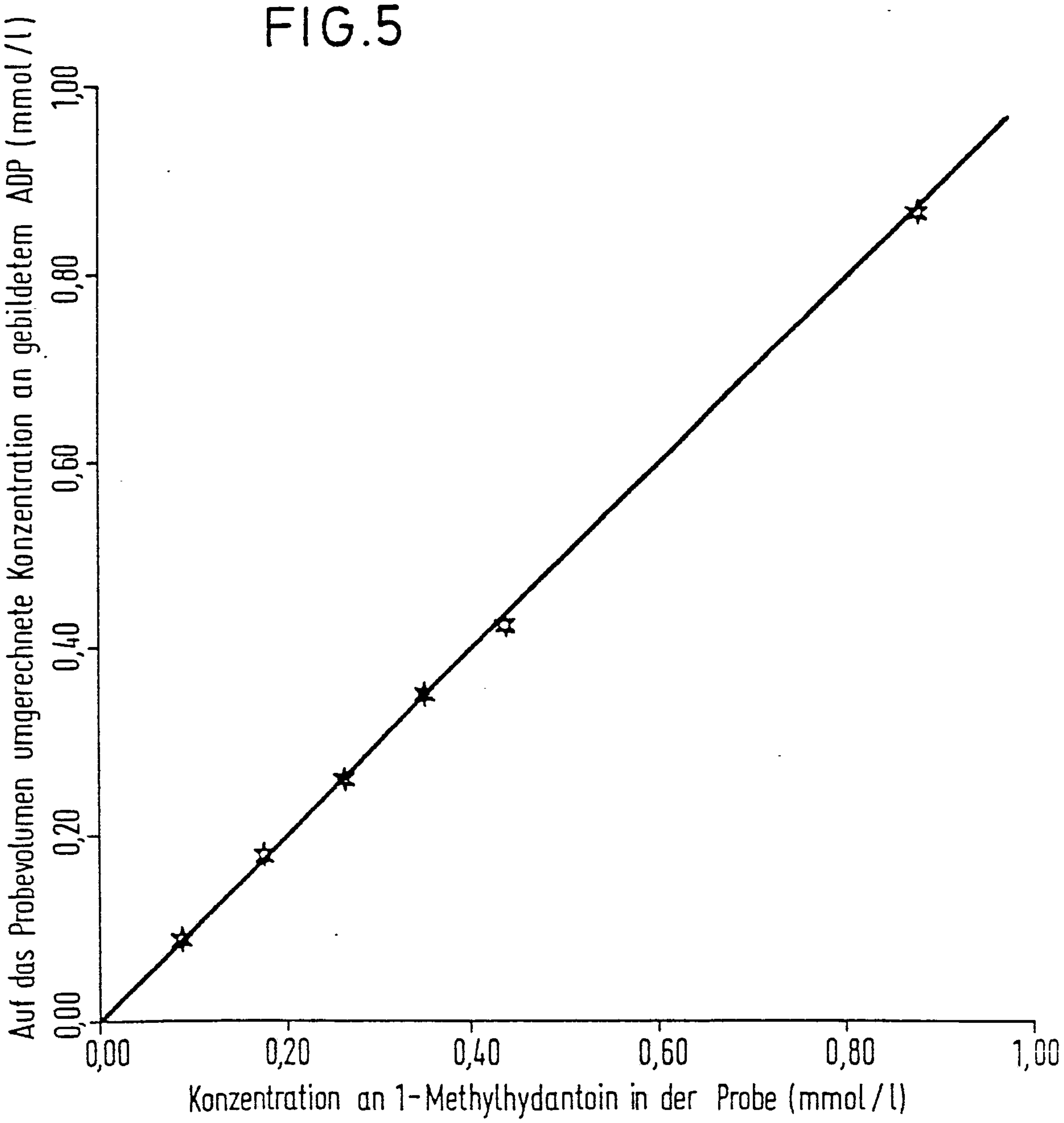
Auf das Probevolumen umgerechnete Konzentration an gebildetem ADP (mmol/l)
1,00
0,80
0,60
0,40
0,20
0,00
0,00
0,20
0,40
0,60
0,80
1,00
Konzentration an 1-Methylhydantoin in der Probe (mmol/l)

FIG. 6

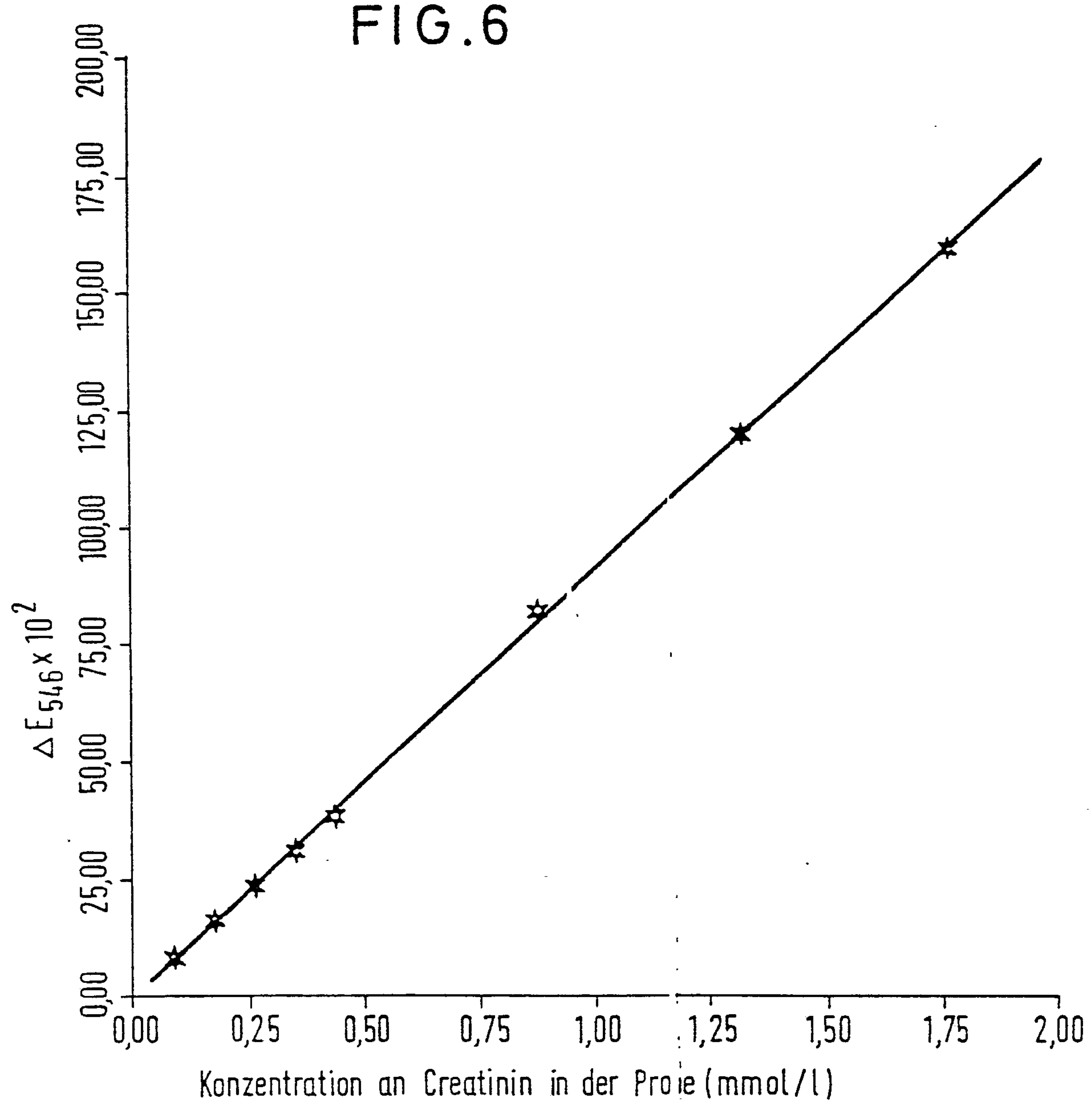
200,00
175,00
150,00
125,00
100,00
75,00
50,00
25,00
0,00
$\Delta E_{546} \times 10^2$
0,00
0,25
0,50
0,75
1,00
1,25
1,50
1,75
2,00
Konzentration an Creatinin in der Pro e (mmol/l)